# EUROPÄISCHE PATENTSCHRIFT

(11) **EP 1 086 199 B1**
(45) Veröffentlichungstag und Bekanntmachung des Hinweises auf die Patenterteilung: **29.05.2002**
(21) Anmeldenummer: 99927871.6
(22) Anmeldetag: 04.06.1999
(51) Int. Cl.: C11D 3/22, C11D 17/00, C11D 1/94, C11D 3/37

(54) **HAFTENDES SANITÄRMITTEL**
ADHESIVE SANITARY AGENT
PRODUIT D'ASSAINISSEMENT ADHESIF

(30) Priorität: 12.06.1998 DE 19826293
(43) Veröffentlichungstag der Anmeldung: 28.03.2001
(73) Patentinhaber: Buck-Chemie GmbH ., 71083 Herrenberg (DE)
(72) Erfinder: DETTINGER, Johannes, D-71260 Horb (DE); JAESCHKE, Edgar, D-70794 Filderstadt (DE); SEIDEL, Detlef, D-73734 Esslingen (DE)
(74) Vertreter: Mammel, Ulrike
(86) Internationale Anmeldenummer: EP9903866
(87) Internationale Veröffentlichungsnummer: WO9966017

(56) Entgegenhaltungen:
- EP-A- 0 589 761
- WO-A-95/18209
- WO-A-95/21239
- WO-A-97/04061
- WO-A-97/40133
- WO-A-98/46712
- CA-A- 1 151 501
- US-A- 3 578 499
- US-A- 5 460 742

## Beschreibung

Die Erfindung betrifft ein Sanitärmittel zum Reinigen und/oder Desinfizieren und/oder zur Duftstoffabgabe für Sanitärgegenstände, wie Toilettenspülbecken.
Es ist bekannt, Sanitärmittel über geeignete Einhängvorrichtungen in den Spülwasserkasten einzuhängen, wobei von dort aus dann die Abgabe des Sanitärmittels gleichfalls bei jedem Spülvorgang in das Toilettenbecken (WC-Becken) erfolgt. Auch sind Sanitärmittel zur Verwendung in WC-Körbchen bekannt, die über einen Halter an dem Toilettenbeckenrand befestigbar sind, wobei das Sanitärmittel bei jedem Spülvorgang das Mittel abgibt.
Nachteilig bei diesen bekannten Vorrichtungen ist das Eintauschen eines neuen Sanitärmittels, beispielsweise in Form eines WC-Reinigungsblockes in das bestehende Aufnahmesystem von Hand, sofern das bisher eingebrachte Sanitärmittel aufgebraucht ist. Bei den Verbrauchern besteht eine psychologische Hemmschwelle, nach Verbrauch des Sanitärmittels ein neues in die jeweilige Vorrichtung einzusetzen, insbesondere bei den "WC-Körbchen", deren Nachfüllung das Hineingreifen in die WC-Schüssel mit der Hand erfordert. Auch sind die dahingehenden Aufnahmevorrichtungen teilweise nur schwer an unhygienischen Stellen zugänglich und ein dahingehender Austauschvorgang ist mit einem gewissen Zeitaufwand verbunden. Das konventionelle WC-Körbchen bereitet ferner aufgrund seines bügelartigen Halters Probleme, wenn man den Toilettenbeckenrand selbst von Hand reinigen möchte, der von dem Halter übergriffen ist.
Nachteilig an den WC-Körbchen ist auch, daß sie - beispielsweise beim Reinigen mit der WC-Bürste - leicht verschoben werden und dann von Hand wieder an die gewünschte Stelle gebracht werden müssen, was unhygienisch ist. Nachteilig ist an diesen WC-Körbchen auch, daß sie von Kindern teilweise als Spielzeug betrachtet und von dem Schüsselrand entfernt werden können.
Üblicherweise wird an einem Sanitärgegenstand ein einziges WC-Körbchen eingesetzt, so daß sowohl die Wirkung des Sanitärmittels örtlich begrenzt, als auch die Dosierung durch das maximale Füllvermögen des Körbchens und/oder die Größe des einzusetzenden Sanitärmittels begrenzt ist.
Solche Sanitärmittel, die in geeigneten Einhängevorrichtungen Verwendung finden, sind in Form fester Blocks und neuerdings auch in Gelform bekannt. Solche gelförmigen Sanitärmittel fassen sich nach Bedarf nachfüllen und nicht erst, wenn das ganze Sanitärmittel vollständig verbraucht ist. Allerdings ist auch bei diesen gelförmigen Sanitärmitteln, die beispielsweise in der DE 197 15 872 beschrieben sind, weiterhin das Befüllen in die Einhängevorrichtung erforderlich, was von den Verbrauchern als unhygienisch betrachtet wird.
Des weiteren sind Sanitärmittel bekannt und auf dem Markt frei erhältlich, die meist flüssig aus Flaschen in das Toilettenbecken von Zeit zu Zeit eingebracht werden, um dann beim nächsten Spülvorgang sofort ab- und ausgespült zu werden. Demgemäß ist für jeden Reinigungs- und/oder Desinfektionsvorgang ein erneuter Auftrag des Sanitärmittels notwendig, was zum einen wiederum mit einem entsprechenden Zeitverbrauch verbunden ist und zum anderen bei entsprechend häufiger, insbesondere bei täglicher Reinigung, zu einem raschen Verbrauch des Sanitärmittels führt. Die mit flüssigen Reinigern erzielte Sauberkeit ist auch nicht anhaltend, und es nimmt nicht jeder sofort ein Sanitärmittel in die Hand, wenn die Toilette einmal verschmutzt ist, sei es im privaten, sei es im öffentlichen Bereich.
In der DE 195 25 604, die flüssige saure Reinigungsmittel zur Entfernung von Kalk betrifft, wird zur Verbesserung der Kalkentfernung - insbesondere an senkrechten Flächen - vorgeschlagen, dem sauren Reinigungsmittel Verdicker zuzusetzen, um die Viskosität zu erhöhen. Allerdings lehrt diese Druckschrift auch, daß sich die Viskosität eines flüssigen Reinigungsmittels nicht beliebig steigern läßt, da sonst die Entfernung von Kalkbelägen und die erforderliche Verteilung des Reinigers nicht mehr erreicht werden kann.
Schwierigkeiten mit einer hygienischen und zeitsparenden Reinigung bestehen auch bei Pissoirs. Diese werden meist mit einem Flüssigkeitsreiniger gesäubert, der jedoch sofort abgespült wird, so daß die Reinigungsspülung und der Geruch nach Frische und Sauberkeit nicht anhalten. Die Pissoirs müssen somit ständig geputzt werden.
Die von den Verbrauchern wegen des erforderlichen Austauschs als unhygienisch empfundene "Körbchenlösung", die jedoch immerhin eine länger anhaltende Reinigung und Beduftung gewährleisten, ist bei Pissoirs in den meisten Fällen wegen der Form des Pissoirs und des Fehlens eines geeigneten Einhängerandes nicht realisierbar. Um eine länger anhaltende Reinigung und Beduftung von Pissoirs zu erzielen, behilft man sich mit "Toilettensteinen", die in die Schüssel beziehungsweise den Abfluß geworfen werden. Diese werden jedoch teilweise vom Spülstrom fortgespült und führen auch zu unerwünschten Spritzern. Auch werden in die Pissoirs teilweise Abfälle wie Zigarettenkippen geworfen, so daß das Reinigungspersonal die Toilettensteine aus den Abfällen in den Pissoirbecken aussortieren muß, sofern die Toilettensteine nicht zusammen mit dem Unrat fortgespült werden.

Ausgehend von diesem Stand der Technik liegt der Erfindung die Aufgabe zugrunde, ein Sanitärmittel zur Verfügung zu stellen, das eine langanhaltende Reinigungs- und/oder Beduftungs- und/oder Desinfektionswirkung erzielt und das auf einfache und hygienische Art und Weise appliziert werden kann.
Eine dahingehende Aufgabe löst ein Sanitärmittel mit den Merkmalen des Anspruchs 1.
Erstaunlicherweise wurde festgestellt, daß es durch Verwendung von Haftvermittlem, nämlich länger- oder langkettigen organischen Molekülen mit wenigstens einem hydrophilen Rest zusammen mit Wasser in dem Sanitärmittel möglich ist, Sanitärmittel bereitzustellen, die direkt auf die Oberfläche des zu reinigenden Sanitärgegenstandes aufgebracht werden können und dort haften, so daß die bekannten, als unhygienisch empfundenen Behältnisse für die Sanitärmittel entfallen können.
Durch die Wechselwirkung zwischen dem hydrophilen Teil der Haftvermittlermoleküle und Wasser wird insbesondere die Oberfläche des Mittels "klebrig", was eine Applikation direkt auf der Oberfläche des Sanitärgegenstands ermöglicht. Die Applikation kann mit jeder geeigneten Vorrichtung wie einer Spritze, einer Tube, einer Kartusche, einer Spraydose etc. erfolgen. Der Verbraucher betätigt nur die Applikationsvorrichtung, ein Kontakt mit einem möglicherweise verunreinigten WC-Körbchen wird vermieden.
Das erfindungsgemäße Mittel kann somit in hygienischer Weise ohne Berührung von mit dem WC-Becken verbundenen, möglicherweise verunreinigten Vorrichtungen aufgebracht und erneuert werden.
Ein wesentlicher Vorteil des erfindungsgemäßen Mittels besteht darin, daß es nach den Wünschen des Verbrauchers portionierbar ist. Wünscht der Verbraucher eine intensivere Beduftung oder wird die Toilette häufig benutzt, so kann entsprechend stärker dosiert werden.
Das erfindungsgemäße Mittel kann auch auf einfache Weise gleichzeitig an verschiedenen Stellen des Sanitärgegenstands appliziert werden, beispielsweise, um sowohl auf der rechten, als auch auf der linken Seite einer Toilettenschüssel eine gleichmäßige Reinigungswirkung zu erzielen.
Auch kann an einem Sanitärgegenstand an verschiedenen Orten das erfindungsgemäße Mittel in unterschiedlichen Zusammensetzungen appliziert werden. Dies ermöglicht beispielsweise, daß zwei untereinander unverträgliche Inhaltsstoffe, beispielsweise halogenfreisetzende Mittel und oxidationsempfindliche Parfümierungsstoffe, durch örtliche Trennung dennoch zu einer gemeinsamen Reinigung und/oder Beduftung der Toilette eingesetzt werden können.
Das Mittel kann entweder durch einen bestimmten Wassergehalt bereits in der zu applizierenden Formulierung "klebrig" sein oder das Haften kann durch eine leichte Befeuchtung der Oberfläche - beispielsweise durch Betätigung der Wasserspülung - und anschließendem Aufbringen des Mittels erreicht werden.
Die erreichte Haftung ist an dem Sanitärgegenstand selbst bei einer Anbringung an einer senkrechten Fläche so gut, daß sich das Mittel sogar unter der zusätzlichen Krafteinwirkung von Spülwasserströmen nicht ablöst.
Infolge des hydrophilen Teils des Haftvermittlers löst sich der Haftvermittler zusammen mit dem restlichen Sanitärmittel nach und nach auf, so daß auch keine unerwünschten "Klebestellen" auf dem Sanitärgegenstand verbleiben, wie es beispielsweise bei einem einfachen "Ankleben" des Sanitärmittels mit doppelseitigem Klebeband der Fall wäre.
Der Haftvermittler bewirkt jedoch nicht nur die Klebeverbindung zusammen mit Wasser, sondern bildet meist auch netzwerkartige Strukturen aus, die dem Mittel, selbst unter der starken Krafteinwirkung durch Spülwasser, die erforderliche Formbeständigkeit verleihen.
Es wird vermutet, daß durch die netzwerkartige Struktur, die die Haftvermittlermoleküle ausbilden, auch eine Verringerung der Auflösegeschwindigkeit des Mittels bewirkt wird, so daß das Mittel erst nach einer größeren Anzahl von Spülvorgängen von dem Sanitärgegenstand abgespült wird. Damit weist das Mittel hohe Standzeiten auf, und zwar ohne daß die bekannten Abgabevorrichtungen in Form der WC-Körbchen oder die Behälter für die Spülwasserkästen erforderlich sind und ohne die zeitaufwendigen und mit Überwindung verbundenen Austauschvorgänge. Aufgrund der verlängerten Standzeit und der damit einhergehenden hohen Anzahl an möglichen Spülvorgängen ist darüber hinaus nur in größeren Zeitabständen ein Neueintrag des Sanitärmittels notwendig, was wiederum Zeit spart.
Die Moleküle des Haftvermittlers sind länger- oder langkettige, im wesentlichen gestreckte Moleküle, die wenigstens teilweise hydrophil sind und somit wenigstens einen hydrophilen Rest oder eine hydrophile Gruppe umfassen, damit die Wechselwirkung mit den Wassermolekülen zur Ausbildung von "Klebestellen" stattfindet. Vorzugsweise sollte es sich bei den Haftvermittlern um unverzweigte Moleküle handeln, um die gewünschte Netzwerkbildung zu ermöglichen.

Der Haftvermittler kann entweder insgesamt hydrophil sein oder auch teilweise hydrophil, teilweise hydrophob.
Als rein hydrophile Haftvermittler können Cellulosen, insbesondere Natrium-Carboxymethylcellulosen, Hydroxyethylcellulosen, Hydroxypropylcellulose oder auch Polysaccharide wie Agar-Agar, Gummi Arabicum, Johannisbrotkernmehl oder Stärke eingesetzt werden. Polysaccharide können schon in Konzentrationen unter 10 Gew.%, unter 5 Gew.% und teils bereits zwischen 1 und 2 Gew.% Netzwerke mit der erforderlichen Festigkeit und einem hinreichenden Klebevermögen ausbilden.
Die Haftvermittlermoleküle können synthetische oder natürliche Polymere, beispielsweise Polyacrylate, Polysaccharide, Polyvinylalkohole oder Polyvinylpyrrolidon sein.
Es ist ebenso möglich, als Haftvermittler Alginate, Diurethane, Gelatine, Pectine, Oleylamine oder Alkyldimethylaminoxide zu verwenden. Auch können als Haftvermittler Polyalkoxyalkane eingesetzt werden. Als hydrophile Reste werden Polyethoxy-, Polypropoxy- oder Polybutoxy- oder auch gemischte Polyalkoxygruppen wie beispielsweise Poly(ethoxypropoxy)gruppen eingesetzt. Besonders bevorzugt ist, als hydrophiles Ende einen Polyethoxyrest, umfassend zwischen 15 und 55 Ethoxygruppen, vorzugsweise zwischen 25 und 45 und besonders bevorzugt 35 +/- 5 Ethoxygruppen, zu verwenden.
Auch Natriumdodecylsulfat, sowie Stearate, insbesondere Natrium oder Kaliumstearat, sind als Haftvermittler geeignet.

Sofern die Haftvermittlermoleküle auch ein hydrophobes Ende aufweisen, sind für den hydrophoben Rest insbesondere geradkettige Alkylreste geeignet, wobei insbesondere geradzahlige Alkylreste wegen der besseren biologischen Abbaubarkeit besonders bevorzugt sind. Um die gewünschte Netzwerkbildung der Haftvermittlermoleküle zu erreichen, sollten die Moleküle unverzweigt sein.
Werden als hydrophobe Reste Alkylgruppen ausgewählt, so sind Alkylreste, mit wenigstens zwölf Kohlenstoffatomen bevorzugt. Besonders gute Ergebnisse wurden mit einer Alkylkettenlänge zwischen 16 und 30 Kohlenstoffatomen, insbesondere 20 und 22 Kohlenstoffatomen, erzielt.
Bevorzugte Haftvermittler sind Polyalkoxyalkane, vorzugsweise ein Gemisch aus C₂₀ bis C₂₂-Alkylethoxylat mit 35 EO, aber auch Natriumdodecylbenzolsulfonat.
Mit Verringerung der Anzahl der Alkoxygruppen wird der Haftvermittler lipophiler, wodurch beispielsweise die Löslichkeit von Parfum und damit die Intensität der Beduftung erhöht werden kann.

Die einzusetzende Konzentration des Haftvermittlers ist von dessen Hydrophilie und dessen Netzwerkbildungsvermögen abhängig. Beim Einsatz von Polysacchariden können beispielsweise schon Konzentrationen zwischen 1 und 2 Gew.% des Haftvermittlers ausreichend sein, wohingegen beim Einsatz von Polyalkoxyalkanen die Konzentrationen zwischen 10 und 40 Gew.%, vorzugsweise zwischen 15 und 35 Gew.% und besonders bevorzugt zwischen 20 und 25 Gew.% betragen können.
Um mit den Haftvermittlermolekülen durch Anlagerung von Wasser die gewünschte Anzahl von Klebestellen bereitzustellen, sollte Wasser wenigstens zu 3 Gew.%, vorzugsweise zwischen 3 und 60 Gew.%, in der Formulierung enthalten sein. Der Wasseranteil ist unter anderem von der Hydrophilie des eingesetzten Haftvermittlers abhängig.
Neben den erfindungsgemäßen Bestandteilen kann das Sanitärmittel weitere übliche Bestandteile umfassen, beispielsweise Desinfektionsmittel, Konservierungsstoffe, wie zum Beispiels Isothiazolon-Derivate oder Schaumstabilisatoren, wie Kokosfettsäureamidopropylbetain oder Kokosfettsäureamidopropyldimethylaminoxid oder Kokosfettsäuremono/diethanolamid aber auch Farbstoffe und/oder Kalk- oder Urinstein lösende Substanzen, insbesondere Säuren.
Auch ist es möglich, dem erfindungsgemäßen Mittel Olefinsulfonate und/oder Säuremethyltauride als Schäumer mit Selbstreinigungswirkung zuzusetzen.
Der Formulierung können - falls gewünscht - auch Salze, wie beispielsweise Natriumsulfat hinzugegeben werden, um die Auflösegeschwindigkeit zu erhöhen. Der Salzanteil kann bis zu 10 Gew.%, vorzugsweise bis zu 5 Gew.%, betragen.
Die erfindungsgemäßen Sanitärmittel sind erst nach einer größeren Anzahl von Spülvorgängen abspülbar. Die Anzahl der Spülvorgänge richtet sich natürlich nach der Zusammensetzung des jeweiligen Sanitärmittels, der aufgebrachten Menge sowie der Geometrie des aufgebrachten Sanitärmittels.
Die Mittel enthalten auch Duft- und/oder Riechstoffe, um die Raumluft zu verbessern, wobei auch daran gedacht ist, ausschließlich Duft- und Riechstoffmedien derart in eine Toilettenschüssel einzubringen. Das Sanitärmittel läßt sich auch für andere Sanitärgegenstände einsetzen wie Urinale, Pissoirs, aber auch für Handspülbecken oder dergleichen.

Vorzugsweise wird das erfindungsgemäße Mittel in Form von salbenartigen pastösen und/oder cremeartigen Gelen hergestellt, die durch Adhäsion erstaunlich gut an der harten Oberfläche kleben. Die Gele sind im wesentlichen formstabil, so daß sie nicht "herunterlaufen" oder "tropfen". Die Haftung und auch die Form der Gele bleibt trotz der durch die Wasserspülung einwirkenden erheblichen Kräfte (Reibung, Deformation ...) erhalten. Vorzugsweise umfassen diese pastösen Gele etwa 10 Gew.% bis 25 Gew.% Duftstoffe, 1 Gew.% bis 5 Gew.% anionische Tenside, 20 Gew.% bis 30 Gew.% nichtionische Tenside, teils als Haftvermittler, 0,5 Gew.% bis 1 Gew.% Verdicker und 0,1 Gew.% bis 0,5 Gew.% Konservierungsmittel sowie etwa 3 bis 60 Gew.% Wasser.
Vorzugsweise werden α-Olefinsulfonate oder Methyltauride als anionische Tenside, sowie Alkyl(C₂₀,C₂₂)ethoxylat mit 35 EO, vorzugsweise 15 Gew.% bis 25 Gew.%, als Haftvermittler und nichtionisches Tensid, insbesondere 0,1 bis 3 Gew.% Aminoxide, besonders bevorzugt etwa 1 bis 2 Gew.% Kokosfettsäureamidopropyldimethylaminoxid (35 %ig) als Schaumstabilisator und Verdicker eingesetzt. Als weiterer Haftvermittler wird Xanthan-Gum und als Konservierungsmittel können lsothiazolon-Derivate verwendet werden.
Die Gele lassen sich vorzugsweise über Tuben, vergleichbar den Zahnpastatuben. Spritzen oder Kartuschen in das Toilettenbecken einbringen und bleiben dort für eine Vielzahl von aufeinanderfolgenden Spülvorgängen haften.
Die an einem Haake-Viskosimeter bestimmte Viskosität dieser pastösen Gele beträgt wenigstens 15000 mPas, üblicherweise wenigstens 25000 mPas, vorzugsweise wenigstens 35000 mPas und besonders bevorzugt wenigstens 60000 mPas.

Es ist ebenfalls möglich, die Sanitärmittel als duftabgebende und/oder reinigende und/oder desinfizierende rigide Gele auszubilden, die vorzugsweise anionische Tenside, Haftvermittler, amphotere Tenside, Glykole und Derivate, Wasser, Parfüm und Konservierungsmittel umfassen. Die rigiden Gele sind im wesentlichen schnittfest und können mittels einer Applikationsvorrichtung, die vorzugsweise auch ein Abschneiden des rigiden Mittels auf die gewünschte Länge ermöglicht, an die befeuchtete oder. trockene Oberfläche geklebt werden. Die schnittfesten Gele können in Form von Scheiben, Blöcken oder sonstigen trennbaren Stücken hergestellt und aufgebracht werden. Die mit einem Haake-Viskosimeter gemessene Viskosität der rigiden Gele sollte vorzugsweise wenigstens 50000 mPas, besonders bevorzugt wenigstens 150000 mPas, betragen.
Die Sanitärmittel können auch thixotrope Flüssigkeiten oder sich auf sonstige Weise nach dem Applizieren verfestigende Flüssigkeiten sein, wobei die Viskosität des Mittels dann nach der Applikation wenigstens 15000 mPas beträgt.
Sofern eine weitere Erhöhung der Standzeit erreicht werden soll, können dem Sanitärmittel zusätzlich Verdickermittel, wie beispielsweise Silikate, zugegeben werden.

Prinzipiell ist es ebenfalls möglich, das Mittel im wesentlichen als reines Beduftungsmittel vorzusehen, das dann an eine Oberfläche "geklebt" wird. Einsatzmittel wären beispielsweise Autos, Mülleimer etc.
Das erfindungsgemäße Mittel wird üblicherweise durch Wirken der Bestandteile mit Wasser hergestellt.

Im folgenden wird das erfindungsgemäße Sanitärmittel anhand eines Ausführungsbeispiels und von Vergleichsversuchen näher erläutert.

### 1. Beispielrezeptur für ein Sanitärmittel als pastöses Gel:

| **Bestandteil** | **Gehalt (%)** | **Handelsname/ Lieferant** | **Funktion:** |
|---|---|---|---|
| α-Olefinsulfonat | 1 - 5 | Hostapur OSB (Clariant) | anionisches Tensid |
| Alkyl (C₂₂)-ethoxylat (35 EO) | 20 - 25 | Imbentin V 100 (Dr. W. Kolb AG) | Haftvermittler |
| Kokosamidopropyldimethylaminoxid (35% ig) | 1 - 3 | Rewominox B 204 (Witco Surfactants) | Schaumstabilisator/ Verdicker |
| Xanthan Gum | 0,5 -1 | Rhodopol T (Rhône Poulenc) | Haftvermittler |
| Isothiazolon-Derivate | 0 - 1 | Parmetol K 40 (Schülke & Mayr) | Konservierungsmittel |
| Wasser | 3 - 60 | | |
| Parfüm | 15-25 | Citrix II 98-1103 (Quest Internat.) | Duftabgabe |

### 2. Vergleichsversuche

Ein pastöses Gel gemäß obiger Rezeptur 1 wurde mit zwei herkömmlichen Sanitärreinigern hinsichtlich des Verhaltens beim Auftragen auf die Porzellanoberfläche, der Haftung und der Standzeit in Abhängigkeit von der Zahl der Spülungen verglichen:
Aus einer Tube mit einer Düsenöffnung von 6 x 26 mm wurden 10 ml der drei Sanitärmittel auf eine WC-Schüssel knapp unter der Spülrinne aufgetragen.

| | Verhalten beim Auftragen auf die Oberfläche | Verhalten bei Betätigung der Spülung |
|---|---|---|
| Gelförmiges pastöses Sanitärmittel gemäß Ausführungsbeispiel 1 | Haftet als rechteckige Raupe an | Verbraucht sich je nach Formulierung in 100 bis 200 Spülungen |
| Flasche 00-WC-Reiniger mit pyrogener Kieselsäure (Firma Yankee Polish); Rezeptur gemäß DE 195 25 604 A1 | Da flüssig, läßt er sich über die Düse nicht dosieren; verteilt sich wie eine Flüssigkeit über die Toilettenschüssel | Nach zwei Spülungen haftet von dem Reiniger nichts mehr an der Porzellanoberfläche |
| WC-Frisch-Gel (Firma Henkel) Rezeptur gemäß DE 197 15 872 A | Läuft sofort in unschönen Schlieren in Richtung Toilettenstumpf | Nach drei Spülungen sind noch etwa 20 % auf der Porzellanoberfläche zu finden, nach fünf Spülungen ist das Gel vollständig fortgespült |

## Patentansprüche

1. Sanitärmittel zum Reinigen und/oder Desinfizieren und/oder zur Duftstoffabgabe, welches Mittel unmittelbar auf dem Sanitärgegenstand applizierbar ist, dort haftet und erst nach einer größeren Anzahl von Spülvorgängen abspülbar ist und
- Wasser,
- anionische und/oder nichtionische und/oder amphotere Tenside,
- Duftstoffe
- einen Haftvermittler,
- sowie ggf. weitere übliche Bestandteile wie Verdicker, Farbstoffe, Konservierungsstoffe umfaßt,
- der Haftvermittler aus der aus Polyalkoxyalkane, Cellulosen, Stärke, Alginaten, Diurethanen, Gelatine, Pectinen, Oleylaminen, Alkyldimethyl aminoxiden, Stearaten, Natriumdodecylbenzolsulfonat, Agar-Agar, Gummi Arabicum, Johannisbrotkernmehl, Polyacrylat, Polyvinylalkohol und Polyvinylpyrrolidon bestehenden Gruppe ausgewählt wird,
- wobei die Polyalkoxygruppe der Polyalkoxyalkane vorzugsweise eine Polyethoxy-, Polypropoxy- oder Polybutoxy- oder auch eine gemischte Polyalkoxygruppe wie eine Poly(ethoxypropoxy)gruppe ist, wobei Polyethoxygruppen mit zwischen 15 und 55 Ethoxygruppen, vorzugsweise zwischen 25 und 45 und besonders bevorzugt 35+/-5 Ethoxygruppen, bevorzugt sind
- und die Viskosität des Mittels wenigstens 15000 mPas beträgt
- das Mittel ein festes oder pastöses Sanitärmittel ist.

2. Sanitärmittel gemäß Anspruch 1, **dadurch gekennzeichnet, daß** die Cellulosen aus der aus Natrium-Carboxymethylcellulosen, Hydroxyethylcellulosen und Hydroxypropylcellulose bestehenden Gruppe und die Stearate aus der aus Natrium- und Kaliumstearat bestehenden Gruppe ausgewählt werden.

3. Sanitärmittel gemäß Anspruch 1, **dadurch gekennzeichnet, daß** unter den Haftvermittlem, die ein hydrophobes Ende umfassen, der hydrophobe Teil des Haftvermittlers aus der Gruppe der Alkylreste, vorzugsweise der unverzweigten Alkylreste, ausgewählt wird und besonders bevorzugt eine geradzahlige Anzahl von Kohlenstoffatomen aufweist.

4. Sanitärmittel nach Anspruch 3, **dadurch gekennzeichnet, daß** der Alkylrest wenigstens zwölf Kohlenstoffatome, vorzugsweise zwischen 16 und 30 Kohlenstoffatomen, insbesondere 20 und/oder 22 Kohlenstoffatome, aufweist.

5. Sanitärmittel nach Anspruch 1, 2, 3 oder 4, **dadurch gekennzeichnet, daß** die Konzentration des Haftvermittlers zwischen 10 und 40 Gew.%, vorzugsweise zwischen 15 und 35 Gew.% und besonders bevorzugt zwischen 20 und 25 Gew.%, beträgt.

6. Sanitärmittel nach Anspruch 1, **dadurch gekennzeichnet, daß** es in Form eines Gels vorliegt.

7. Sanitärmittel nach einem der Ansprüche 1 bis 6, **dadurch gekennzeichnet, daß** die Viskosität wenigstens 25.000 mPas, vorzugsweise wenigstens 35.000 mPas und besonders bevorzugt wenigstens 60.000 mPas, beträgt.

8. Sanitärmittel nach Anspruch 1, **dadurch gekennzeichnet, daß** das Sanitärmittel ein rigides Gel ist und Glykole und/oder Glykolderivate umfaßt.

9. Verfahren zur Applikation eines Sanitärmittels nach einem der Ansprüche 1 bis 6, **dadurch gekennzeichnet, daß** das Mittel direkt auf die Oberfläche des zu reinigenden Sanitärgegenstandes aufgebracht wird und dort haftet.

## Claims

1. Sanitary agent for cleaning and/or disinfecting and/or releasing a deodorant, which agent can be applied directly to the sanitary object, that adheres there and which agent is washed away only after a large number of rinse actions, which sanitary agent comprises
- water,
- anionic and/or non-ionic and/or amphoteric tensides,
- fragrances,
- an adhesion promoter,
- and optional other usual components such as thickeners, colorants, preservatives,
- whereby the adhesion promoter is chosen from the group of the polyalkoxy alkanes, cellulose, starch, alginates, diurethanes, gelatines, pectines, oleyl amines, alkyl dimethyl amine oxides, stearates, sodium dodecylbenzene sulfonates, agar agar, acacia gum, carob bean flour, polyacrylates, polyvinyl alcohols and polyvinyl pyrrolidones,
- the polyalkoxy group of the polyalkoxy alkanes is preferably a polyethoxy, polypropoxy or polybutoxy or also a mixed polyalkoxy group such as a poly(ethoxypropoxy) group, whereby polyethoxy groups with between 15 and 55 ethoxy groups are preferred, preferably between 25 and 45, and especially preferred 35 +/- 5 ethoxy groups,
- the viscosity of the agent is at least 15.000 mPas,
- the agent is a pasty or firm sanitary agent.

2. Sanitary agent according to claim 1, wherein the cellulose is selected from the group consisting of sodium carboxymethyl cellulose, hydroxyethyl cellulose and hydroxypropyl cellulose and the stearates are selected from the group consisting of sodium and potassium stearate.

3. Sanitary agent according to claim 1, wherein for the adhesion promoters having a hydrophobic end the hydrophobic part of the adhesion promoter is selected from the group consisting of alkyl residuals, preferably the unbranched alkyl residues, and especially preferred with an even-numbered number of carbon atoms.

4. Sanitary agent according to claim 3, wherein the alkyl residual has at least twelve carbon atoms, preferably between 16 and 30 carbon atoms, especially 20 and/or 22 carbon atoms.

5. Sanitary agent according to claim 1, 2, 3 or 4, wherein the concentration of the adhesion promoter is between 10 and 40 percentage by weight, preferably between 15 and 35 percentage by weight, and especially preferred between 20 and 25 percentage by weight.

6. Sanitary agent according to claim 1 in the form of a gel.

7. Sanitary agent according to claim 1 to 6, wherein the viscosity is at least 25.000 mPas, preferably at least 35.000 mPas, and especially preferred at least 60.000 mPas.

8. Sanitary agent according to claim 1, **characterized by** the fact that it is a rigid gel and includes glycols and/or glycol derivates.

9. Process for application of a sanitary agent according to one of claims 1 to 6, **characterized by** the fact that the agent is applied directly to the surface of the sanitary object to be cleaned and adheres thereto.

## Revendications

1. Produit sanitaire pour le nettoyage et/ou la désinfection et/ou la libération de parfum, lequel produit peut être appliqué directement sur l'appareil sanitaire, y adhère et n'est éliminé qu'après un plus grand nombre de chasses, et comprend
- de l'eau,
- des tensioactifs anioniques et/ou non ioniques et/ou amphotères,
- des parfums,
- un promoteur d'adhérence,
- ainsi qu'éventuellement d'autres composants usuels tels qu'épaississants, colorants, conservateurs,
- le promoteur d'adhérence est choisi dans le groupe constitué par des polyalcoxyalcanes, celluloses, l'amidon, des alginates, diuréthannes, gélatines, pectines, oléylamines, oxydes d'alkyldiméthylamines, stéarates, le dodécylbenzènesulfonate de sodium, l'agar-agar, la gomme arabique, la farine de caroube, le polyacrylate, le poly(alcool vinylique) et la polyvinylpyrrolidone,
- le groupe polyalcoxy des polyalcoxyalcanes étant de préférence un groupe polyéthoxy, polypropoxy ou polybutoxy ou bien un groupe polyalcoxy mixte, tel qu'un groupe poly(éthoxypropoxy), les groupes polyéthoxy comportant entre 15 et 55 groupes d'éthoxy, de préférence entre 25 et 45 et, de façon particulièrement préférée, 35 ± 5 groupes éthoxy étant préférés,
- et la viscosité du produit est d'au moins 15 000 mPa.s,
- le produit est un produit sanitaire solide ou pâteux.

2. Produit sanitaire selon la revendication 1, **caractérisé en ce que** les celluloses sont choisies dans le groupe constitué par les carboxyméthylcelluloses sodiques, les hydroxyéthylcelluloses et l'hydroxypropylcellulose, et les stéarates sont choisis dans le groupe constitué par le stéarate de sodium et le stéarate de potassium.

3. Produit sanitaire selon la revendication 1, **caractérisé en ce que** parmi les promoteurs d'adhérence qui comprennent une extrémité hydrophobe, on choisit la partie hydrophobe du promoteur d'adhérence dans le groupe des radicaux alkyle, de préférence des radicaux alkyle non ramifiés, et, de façon particulièrement préférée, comprenant un nombre pair d'atomes de carbone.

4. Produit sanitaire selon la revendication 3, **caractérisé en ce que** le radical alkyle comprend au moins douze atomes de carbone, de préférence entre 16 et 30 atomes de carbone, en particulier 20 et/ou 22 atomes de carbone.

5. Produit sanitaire selon la revendication 1, 2, 3 ou 4, **caractérisé en ce que** la concentration du promoteur d'adhérence est comprise entre 10 et 40 % en poids, de préférence entre 15 et 35 % en poids, et, de façon particulièrement préférée, entre 20 et 25 % en poids.

6. Produit sanitaire selon la revendication 1, **caractérisé en ce qu'**il se trouve sous forme d'un gel.

7. Produit sanitaire selon l'une quelconque des revendications 1 à 6, **caractérisé en ce que** la viscosité est d'au moins 25 000 mPa.s, de préférence d'au moins 35 000 mPa.s et, de façon particulièrement préférée, d'au moins 60 000 mPa.s.

8. Produit sanitaire selon la revendication 1, **caractérisé en ce que** le produit sanitaire est un gel rigide et comprend des glycols et/ou des dérivés de glycols.

9. Procédé pour l'application d'un produit sanitaire selon l'une quelconque des revendications 1 à 6, **caractérisé en ce que** le produit est appliqué directement sur la surface de l'appareil sanitaire à nettoyer, et y adhère.
